(19) Europäisches Patentamt / European Patent Office / Office européen des brevets

(11) **EP 2 745 770 B1**

(12) **EUROPÄISCHE PATENTSCHRIFT**

(45) Veröffentlichungstag und Bekanntmachung des Hinweises auf die Patenterteilung:
**08.07.2020   Patentblatt 2020/28**

(51) Int Cl.:
**A61B 5/024** *(2006.01)*        **A61B 5/0402** *(2006.01)*

(21) Anmeldenummer: **13196722.6**

(22) Anmeldetag: **11.12.2013**

(54) **Verfahren und Vorrichtung zur Bestimmung der Herzfrequenzvariabilität eines Lebewesens**

Method and device for determining the variability of a creature's heart rate

Procédé et dispositif de mesure de la variabilité de la fréquence cardiaque d'un être vivant

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.12.2012   DE 102012112555**

(43) Veröffentlichungstag der Anmeldung:
**25.06.2014   Patentblatt 2014/26**

(73) Patentinhaber: **Wittling, Ralf Arne**
**66540 Neunkirchen (DE)**

(72) Erfinder:
• **WITTLING, Werner**
**66540 Neunkirchen (DE)**
• **WITTLING, Arne**
**66540 Neukirchen (DE)**

(74) Vertreter: **Wolff, Matthias et al**
**Patentanwälte Bernhardt/Wolff**
**Partnerschaft mbB**
**Europaallee 17**
**66113 Saarbrücken (DE)**

(56) Entgegenhaltungen:
**US-A1- 2009 005 696      US-A1- 2010 042 172**
**US-A1- 2011 105 926**

**Beschreibung**

Die Erfindung betrifft ein Verfahren zur Bestimmung der Herzfrequenzvariabilität eines Lebewesens, insbesondere eines Menschen, bei dem mittels einer Messeinrichtung Herzschläge des Lebewesens und Intervalle zwischen den Herzschlägen in Abhängigkeit von der Zeit als ein Messergebnis bestimmt werden, die Intervalle mittels schneller Fourier-Transformation (Fast Fourier Transformation) analysiert werden und ein Frequenzspektrum erstellt wird, bei dem vor Analyse mittels der Fourier-Transformation eine Prüfung der bestimmten Intervalle durchgeführt wird und das Messergebnis in Abhängigkeit von einem Ergebnis der Prüfung bearbeitet wird, wobei bei der Prüfung der ermittelten Intervalle geprüft wird, ob die Herzschläge zur Bestimmung der Herzfrequenzvariabilität geeignet sind, und zumindest einer der Herzschläge, die zur Bestimmung der Herzfrequenzvariabilität ungeeignet sind, aus dem Messergebnis gelöscht wird und durch einen künstlichen Herzschlag ersetzt wird, der im Messergebnis eine aufgrund zumindest einer der Herzschläge vor und nach dem ungeeigneten Herzschlag zu erwartende zeitliche Anordnung aufweist.

Die Erfindung betrifft ferner eine Vorrichtung zur Durchführung des Verfahrens.

Ein solches Verfahren, bei dem über einen Zeitraum von mehreren Minuten ein EKG einer Person erstellt wird, ist aus der WO 2011/113428 A2 der Anmelder bekannt.

Aus der US 2009/0005696 A1 ist ein Verfahren bekannt, bei dem die Aktivität des autonomen Nervensystems eines Patienten mittels einer Untersuchung der Herzfrequenzvariabilität bestimmt wird. Dabei werden bei einer Messung der Herzfrequenz mittels EKG fehlerhafte Messwerte korrigiert, bevor eine FourierAnalyse des Messsignals durchgeführt wird. Mit Hilfe eines sog. "clusterization algorithm" werden anschließend Werte für das parasympathische Nervensystem und solche für das sympathische Nervensystem bestimmt, so dass ein Verhältnis der Intensitäten dieser beiden Werte zur Analyse des Ergebnisses grafisch aufgetragen werden kann.

Aus der US 2010/0042172 A1 und der US 2011/0105926 A1 sind Verfahren zur Bestimmung der Herzfrequenzvariabilität bekannt, bei denen nach der Bestimmung der Messwerte des EKG's eine Fehlerkorrektur durch Ersetzen fehlerhafter Signale oder Artefakte durchgeführt wird, bevor das Messsignal einer Fourieranalyse unterzogen und danach ausgewertet wird.

Der körperliche Zustand eines Lebewesens, z.B. seine Gesundheit, Fitness oder ein Stresszustand, wird u.a. durch sein Vermögen bestimmt, sich an Belastungen anzupassen und diesen standzuhalten. Eine solche Anpassung erfolgt über das autonome Nervensystem, das u.a. die Schlagfrequenz, Atmung, Blutdruck, Verdauung und Stoffwechsel abhängig von der jeweiligen Belastung reguliert. Ein Zweig des autonomen Nervensystems, dessen Aktivierung Leistungs- und Reaktionsbereitschaft und die Aktivierung von Energiereserven fördert, wird als Sympathikus bezeichnet. Die Aktivierung eines weiteren, als Gegenspieler zum Sympathikus wirkenden Zweigs des autonomen Nervensystems fördert Ruhe und Erholung sowie Energiespeicherung und wird Parasympathikus genannt. Es ist aus zahlreichen Studien bekannt, dass die Aktivität von Sympathikus und Parasympathikus zur Beurteilung einer Leistungsfähigkeit von Menschen dienen kann. Die Auswirkungen von Aktivitäten des Sympathikus oder Parasympathikus lassen sich z.B. anhand von Schwankungen der zeitlichen Abstände zwischen den Herzschlägen bestimmen.
Ein niederfrequenter Bereich des mittels der schnellen Fourier-Transformation ermittleten Frequenzspektrums, nämlich 0,04 bis 0,15 Hz, lässt sich der Aktivität des Sympathikus zuordnen, da eine Beeinflussung des Herzschlages durch den Sympathikus in einem verhältnismäßig langsamen Rhythmus erfolgt. Ein Bereich höherer Frequenz, nämlich 0,15 bis 0,4 Hz, ist einer Aktivität des Parasympathikus zuzuordnen, der den Herzschlag in einem schnelleren Rhythmus beeinflusst. Durch Auswertung des Frequenzspektrums lassen sich die Aktivitäten des Sympathikus und des Parasympathikus im Verhältnis zueinander bestimmen und es können Rückschlüsse auf körperliches und geistiges Wohlergehen des Lebewesens geschlossen werden.

Problematisch ist, dass sowohl bei Erstellung des Messergebnisses als auch bei Analyse des Messergebnisses Fehler und Ungenauigkeiten auftreten können, die das ermittelte Frequenzspektrum stark verändern können und deshalb eine korrekte Bestimmung der Herzfrequenzvariabilität verhindern.

Der Erfindung liegt die Aufgabe zugrunde, ein Verfahren der eingangs genannten Art zu schaffen, mit dem sich die Herzfrequenzvariabilität genauer bestimmen lässt.

Erfindungsgemäß wird diese Aufgabe dadurch gelöst, dass die zeitliche Anordnung des künstlichen Herzschlags davon abhängig vorgesehen wird, ob der ungeeignete Herzschlag zu einem Zeitpunkt stattgefunden hat, zu dem die Frequenz der Herzschläge wegen respiratorischer Sinusarrhythmie (RSA) steigend oder sinkend war.

Insbesondere aus organischen Gründen, z.B. Herzrhythmusstörungen, oder aus messtechnischen Gründen, ggf. als Folge von Bewegungen des Körpers des Lebewesens (z.B. beim Husten) bei Erstellung des Messergebnisses, sind im Messergebnis oftmals ungenaue oder fehlerhafte Signale zu finden, welche die Bestimmung der Herzfrequenzvariabilität stark stören können. Die dadurch verursachten Ungenauigkeiten und Fehler lassen sich beheben, wenn das Elektrokardiogramm vor Durchführung der Fourier-Transformation geprüft wird und die Ungenauigkeiten und die Fehler im Messergebnis behoben werden. Deshalb wird nach Erstellung des Messergebnisses zunächst geprüft, ob sich in dem Messergebnis ein Herzschlag findet, der zur Bestimmung der Herzfrequenzvariabilität ungeeignet ist. Wird ein solcher Ausschlag gefunden, wird ein Abschnitt, der den Herzschlag aufweist, aus dem EKG gelöscht und im Messergebnis durch einen Abschnitt ersetzt, der einen künstlichen Herzschlag aufweist, der im Messergebnis eine aufgrund zumindest einer der Herzschläge vor und nach dem ungeeigneten Herzschlag zu erwartende zeitliche Anordnung, vorzugsweise einen Zeitpunkt und eine Dauer, aufweist. Dabei wird eine Anordnung des künstlichen Herzschlags abhängig davon vorgesehen, ob der ungeeignete Herzschlag zu einem Zeitpunkt stattgefunden hat, zu dem die Frequenz der Herzschlägen wegen der respiratorischen Sinusarrhythmie (RSA) steigend oder sinkend war. Dies lässt sich anhand der sich ändernden Intervalle aus dem Messergebnis ermitteln.

Vorteilhaft wird durch Ersetzen des Abschnitts vermieden, dass durch den ungeeigneten Herzschlag das Frequenzspektrum beeinflusst wird. Würde der Abschnitt dagegen z.B. einfach aus dem Messergebnis herausgeschnitten und nicht ersetzt, würde zum einen die Gesamtzeit der Messung verkürzt. Ferner würden rhythmische Schwankungen der Intervalle z.B. wegen respiratorischer Sinusarrhythmie (RSA), die zur Folge hat, dass beim Einatmen die Intervalle kürzer und beim Einatmen länger werden, verändert. Dies führte dazu, dass bei Analyse mittels der schnellen Fourier-Transformation nicht physiologisch bedingte Faktoren berücksichtigt würden, insbesondere würden in der Frequenzdomäne nicht physiologisch bedingte Energien in der jeweiligen Frequenz in den betroffenen Frequenzbereich induziert. Dadurch würden die Ungenauigkeiten und Fehler in die Auswertung eingebracht werden und das zu interpretierende Ergebnis würde verfälscht.

Um die genaue Bestimmung der Herzfrequenzvariabilität sicherzustellen, wird der ungeeignete Herzschlag im Messergebnis zweckmäßigerweise durch einen künstlichen Herzschlag ersetzt, der im Messergebnis die aufgrund von zumindest je drei, vorzugsweise zumindest je vier, der Intervalle zwischen den Herzschlägen vor und nach dem ungeeigneten Herzschlag zu erwartende Anordnung aufweist.

Zweckmäßigerweise nimmt der künstliche Herzschlag im Messergebnis einen gleich großen oder im Wesentlichen gleich großen Zeitabschnitt ein wie der fehlerhaft ermittelte Herzschlag.

In einer Ausgestaltung der Erfindung wird die Anordnung des künstlichen Herzschlags mittels Interpolation der Intervalle zwischen den Herzschlägen vor und nach dem ungeeigneten Herzschlag bestimmt.

Bei steigender Frequenz wird der künstliche Herzschlag zwweckmäßigerweise in einem kleineren Intervall zu dem Herzschlag vor dem ungeeigneten Herzschlag als zu dem Herzschlag nach dem ungeeigneten Herzschlag angeordnet. War die Frequenz sinkend, wird umgekehrt der künstliche Herzschlag vorzugsweise in einem größeren Intervall zu dem Herzschlag vor dem ungeeigneten Herzschlag als zu dem Herzschlag nach dem ungeeigneten Herzschlag angeordnet. Fand der Herzschlag zu einem Zeitpunkt statt, zu dem zwischen sinkender und steigender Frequenz gewechselt wird, wird der Herzschlag dementsprechend umgekehrt, d.h. passend zu der sich jeweilig ändernden Frequenz, vorgesehen.

In einer besonders bevorzugten Ausgestaltung der Erfindung wird als Messeinrichtung ein EKG-Gerät verwendet und zur Bestimmung der Herzschläge erste positive Ausschläge einer Herzkammerregung (R-Ausschläge) ermittelt. Die genannten Intervalle sind dann die Intervalle zwischen den Ausschlägen (RR-Intervalle). Die genannte respiratorische Sinusarrhythmie lässt sich ferner anhand der Amplitude der R-Ausschläge bestimmen.

Ferner könnte auch ein Herzfrequenzmessgerät, beispielsweise ein Brustgurt oder ein optischer Sensor, der an einen Finger oder ein Ohrläppchen angelegt wird, verwendet werden.

In einer weiteren Ausgestaltung der Erfindung werden die in Abhängigkeit von der Zeit ermittelten Intervalle zwischen den Herzschlägen durch Neuabtasten in eine Zeitreihe mit gleichgroßen Intervallabständen gewandelt.

Bei den, gegebenenfalls bearbeiteten, Intervallen handelt es sich naturgemäß um eine unregelmäßige Zeitreihe. Es ist es nötig, die Daten durch das Neuabtasten (resampling) in eine regelmäßige Zeitreihe mit gleichgroßen Intervallabständen zu wandeln, weil das Vorliegen einer regelmäßigen Zeitreihe eine Bedingung für spektralanalytische Bestimmung in der Frequenzdomäne (frequency domain) bei der Fast-Fourier-Transformation ist. Es hat sich dabei als besonders vorteilhaft erwiesen, zur Neuabtastung eine auf kubischen Splines basierende Interpolationsmethode anzuwenden.

In einer weiteren Ausgestaltung der Erfindung wird die Neuabtastung derart durchgeführt, dass die Anzahl der Intervalle zwischen den Herzschlägen gleich einer Zweierpotenz ($2^n$) ist. Vorteilhaft kann dann auf ein Zeropadding der Intervalle vor der Analyse mittels der schnellen Fourier-Transformation verzichtet werden. Als Zeropadding bezeichnet man das Auffüllen der Zeitreihe mit Nullen vor der Analyse, für den Fall, dass die Anzahl der er Intervalle zwischen den Herzschlägen ungleich einer Zweierpotenz ist. Ein solches Zeropadding entspräche einer Verlängerung der Messzeit und führte, weil die Messzeit T und die digitale Auflösung 1/T in einem reziproken Zusammenhang stehen, zu einer höheren Auflösung. Nach dem Zeropadding wäre dann zwar der Abstand der Datenpunkte im Frequenzspektrum geringer, dies liefert allerdings keine zusätzlichen Informationen.

Die Anwendung des Zeropadding wird insbesondere dann problematisch, wenn das letzte, wie nachfolgend beschrieben mittels Detrending betarbeitete Intervall ungleich Null ist und die Anzahl der zur Auffüllung der benötigten Intervalle auf die Zweierpotenz mit Nullen so groß ist, dass der Übergang außerhalb des Wirkungsbereiches der nachfolgend beschriebenen Gewichtungsfunktion liegt. Ein derart verursachter künstlicher Sprung außerhalb des Wirkungsbereichs der Gewichtungsfunktion würde bei der schnellen Fourier-Transformation nicht physiologische Anteile erzeugen, welche eine Interpretation des Ergebnisses stark verfälschen könnten.

Zweckmäßigerweise wird von den ermittelten Intervallen zwischen den Herzschlägen ein linearer oder parabolischer Trend subtrahiert (Detrending).

Dazu wird zweckmäßigerweise eine Gerade bzw. Kurve berechnet, die dem mittleren Verlauf des, ggf. bearbeiteten, Messergebnisses folgt. Der lineare Trend berechnet sich mit der Gleichung

$$Y = k \bullet X + d.$$

Die Steigung k der Geraden und der Y-Achsenabschnitt müssen jeweils aus dem Messergebnis bestimmt werden. Durch ein lineares Detrending wird nur der Gleichanteil vom Messergebnis subtrahiert, der dem arithmetischen Mittelwert des Signals entspricht. Dieser Gleichanteil ist reel und hat keine Phase. Er findet sich deshalb nach der Fourieranalyse im 0-ten Element des Ergebnisfeldes wieder und verfälscht zum einen die Interpretation von Frequenzkomponten, die unterhalb des eingangs genannten niederfrequenten Bereichs liegen. Der Gleichanteil wirkt sich wegen des nachfolgend erläuterten Leakage-Effektes zum anderen auch auf die nahen Ergebnisfelder des niederfrequenten Bereichs des Frequenzspektrums aus. Um eine Interpretationsfähige Analyse Herzfrequenzvariabilität zu erhalten, muss der Gleichanteil deswegen mittels des Detrendings eliminiert werden.

Ein parabolischer Trend, der u.a. durch Änderung der Frequenz der Herzkammerregungen hervorgerufen wird, berechnet sich mit folgender quadratischen Funktion:

$$Y = k_0 + k_1 \bullet X + k_2 \bullet X^2$$

Der Parameter $k_0$ beschreibt die Position in Y-Richtung, $k_1$ beschreibt die Position in X- und Y-Richtung und $k_2$ die Stärke und die Richtung der Krümmung.

Weil die schnelle Fourier-Transformation nicht auf instationäre Signale angewendet werden kann, können durch eine sich erhöhende Frequenz der Herzkammerregungen verursachten niederfrequenten Anteile im Messergebnis durch die Eliminierung höhergradiger Trends reduziert werden. Dazu kann z.B. ein Detrending mit orthogonalen Polynomen oder Smooth Blinds mit variablem a-Faktor verwendet werden.

In einer bevorzugten Ausführungsform der Erfindung werden die ermittelten, ggf. wie oben beschriebenen bearbeiteten, Intervalle durch Multiplikation mit einer Funktion zur Gewichtung, vorzugsweise einer Hann-, Hamming-, Kasier-Bessel- oder Gauß-Fensterfunktion, gewichtet werden.

Da bei der schnellen Fourier-Transformation die, ggf. bearbeiteten, Intervalle in Abschnitte unterteilt werden, die immer wieder aneinander gehängt werden, die Abschnitte selbst aber endlich sind, treten im Frequenzspektrum Leckkomponenten (leakage effects) auf, wenn die Länge der Abschnitte nicht ein ganzzahliges Vielfaches der Signalperiode ist. Dies hat zur Folge, dass das errechnete Frequenzspektrum breiter und an den Rändern "verschmierter" ist, als es aufgrund der tatsächlich enthaltenen Frequenzanteile sein dürfte. Durch die geeignete Wahl der Gewichtungsfunktion können Leck-Komponenten reduziert werden. Die Multiplikation mit der Gewichtungsfunktion entspricht einer Faltung des Signals mit dem Spektrum der Gewichtungsfunktion in der Frequenzdomäne.

In einer weiteren Ausgestaltung der Erfindung wird die spektrale Leistungsdichte (power spektrum) des genannten erstellten Frequenzspektrums in einem Bereich von 0 Hz bis 0,4 Hz bestimmt, wobei die spektrale Leistungsdichte das Quadrat des Betrags der Fourier-Transformierten ist

$$(spektrale\ Leistungsdichte = \sqrt{Realteil^2 + Imaginärteil^2}\ ).$$

In einer Ausführungsform der Erfindung wird die spektrale Leistungsdichte separat für den Frequenzbereich von 0,003 bis 0,04 Hz (very low frequency, VLF), von 0,04 bis 0,15 Hz (low frequency, LF), von 0,15 bis 0,4 Hz (high frequency, HF) bestimmt.

Ferner kann die spektrale Leistungsdichte auch für den Frequenzbereich von 0,003 bis 0,4 Hz (total power, TP) bestimmt werden.

Zweckmäßigerweise werden zur Bestimmung der spektralen Leistungsdichte lediglich die zu dem Frequenzbereich gehörenden Intervalle herangezogen.

Da die Ergebnisse der schnellen Fourrier-Transformation nicht kontinuierlich einem einzigen Frequenzbereich zugeordnet werden können, sondern in diskreten Ergebnisbehältern vorliegen, erfolgt die Zuordnung der Ergebnisbehälter derart, dass jeder Grenze der jeweils am nächsten an der Grenze liegende Ergebnisbehälter exklusiv zugeordnet wird.

Die Erfindung wird nachfolgend anhand eines Ausführungsbeispiels und der beigefügten, sich auf diese Ausführungsbeispiel beziehenden Zeichnung näher erläutert.

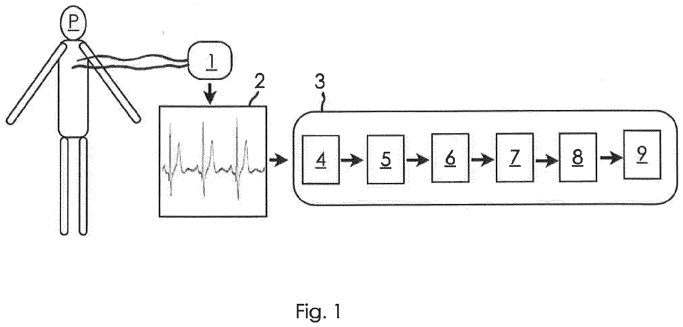

In Fig. 1 ist schematisch eine Person P dargestellt, mit der über Elektroden ein EKG-Gerät 1 verbunden ist. Ein mittels des EKG-Geräts 1 aufgezeichnetes Elektrokardiogramm (EKG) 2 wird an ein Mittel zur Datenverarbeitung 3 übertragen, das zur Durchführung des erfindungsgemäßen Verfahrens eingerichtet ist.

Aus dem EKG 2 werden in einem Verfahrensschritt 4 erste positive Ausschläge der Herzkammererregung (R-Ausschläge) ermittelt und geprüft, ob diese während der Aufnahme des EKGs in einem gewöhnlichen Rhythmus erfolgt sind. Wird bei Überprüfung festgestellt, dass ein arrhythmischer R-Ausschlag gemessen worden ist, wird der R-Ausschlag aus dem Elektrokardiogramm gelöscht und durch einen künstlichen R-Ausschlag ersetzt, der im EKG derart angeordnet wird, wie es im gewöhnlichen Rhythmus zu erwarten gewesen wäre. Die Anordnung des künstlichen R-Ausschlags wird dazu mittels Interpolation der letzten vier R-Ausschläge vor und der vier Ausschläge nach dem arrhythmischen R-Ausschlag bestimmt. Dabei wird berücksichtigt, ob der arrhythmische R-Ausschlag zu einem Zeitpunkt stattgefunden hat, zu dem die Frequenz der R-Ausschläge wegen respiratorischer-Arrhythmie steigend oder sinkend war. War die Frequenz steigend, wird der künstliche R-Ausschlag in einem kleineren Intervall zu dem R-Ausschlag vor dem ungeeigneten R-Ausschlag als zu dem R-Ausschlag nach dem ungeeigneten R-Ausschlag angeordnet. War die Frequenz dagegen sinkend, wird der künstliche R-Ausschlag in einem größeren Intervall zu dem R-Ausschlag vor dem ungeeigneten R-Ausschlag als zu dem R-Ausschlag nach dem ungeeigneten R-Ausschlag angeordnet. Fand der R-Ausschlag zu einem Zeitpunkt statt, zu dem zwischen sinkender und steigender Frequenz gewechselt wird, wird der R-Ausschlag dementsprechend umgekehrt, d.h. passend zu der sich jeweilig ändernden Frequenz, vorgesehen.

Anschließend werden in einem Verfahrensschritt 5 die ermittelten Intervalle (R-R-Zeiten) zwischen den R-Ausschlägen durch Neuabtastung in eine Zeitreihe mit äquidistanten Intervallabständen gewandelt, wobei eine auf kubischen Splines basierende Interpolationsmethode angewendet wird und eine Integrationsbreite derart gewählt wird, dass die Intervalle zwischen den R-Ausschlägen gleich einer Zweierpotenz ist.

In einem Verfahrensschritt 6 wird bestimmt, ob sich im EKG ein Trend findet, der auf eine Änderung der Frequenz der R-Ausschläge zurückzuführen ist und der ggf. aufgefundene Trend zu den ermittelten Intervallen zwischen den R-Ausschlägen abgezogen. Zumindest wird ein linearer Trend zur Entfernung des Gleichanteils abgezogen.

Danach werden in einem Verfahrensschritt 7 die ermittelten Intervalle durch Multiplikation mit einer Hann-Funktion gewichtet.

Mit den nunmehr vorliegenden bearbeiteten Informationen wird in einem Verfahrensschritt 9 mittels schneller Fourier Transformation eine Spektralanalyse durchgeführt. Dazu wird eine spektrale Leistungsdichte (power spectrum) im Frequenzbereich von 0,003 bis 0,04 Hz (very low frequency, VLF), von 0,04 bis 0,15 Hz (low frequency, LF), von 0,15 bis 0,4 Hz (high frequency, HF) bestimmt. Dazu werden getrennt voneinander, insbesondere nicht überlappend, lediglich die zu den jeweiligen Frequenzbereichen gehörenden Intervalle herangezogen. Die spektrale Leistungsdichte wird durch die Unterteilung in die Frequenzbereiche der jeweiligen physiologischen Bedeutung zugeordnet. So ist der LF-Bereich ein Maß für die sympathische Aktivierung und der HF-Bereich ein Maß für die parasympathische Aktivierung. Der Quotient aus LF und HF gibt einen Hinweis auf den körperlichen Zustand der Person P.

Insgesamt lassen sich aus einem in einem Verfahrensschritt 9 bestimmten Ergebnis dieser Spektralanalyse Rückschlüsse auf die Herzfrequenzvariabilität des Patienten schließen.

Nachfolgend wird gezeigt, wie sich die Bearbeitung eines EKGs, das eine zur Bestimmung der Herzratenvariabilität ungeeigneten R-Ausschlag zeigt, auf ein Ergebnis der Analyse mittels der schnellen Fourier-Transformation auswirkt. Dazu wird als Referenz ein EKG herangezogen, das keinen ungeeigneten R-Ausschlag aufweist. Spektralwerte VLF, LF und HF sowie der Quotient LF/HF finden sich in der Spalte "Referenz" in Tabelle 1.

Zur Überprüfung des erfindungsgemäßen Verfahrens wurde in dem EKG künstlich ein einziger R-Ausschlag in Position einer supraventrikulären Extrasystole (SVES) versetzt. Die Spektralwerte dazu finden sich in Tabelle 1 unter "SVES-Referenz".

Unter "SVES-korrigiert-1" finden sich in der Tabelle die Ergebnisse, die bei einer Korrektur eines EKGs erzielt werden, bei dem der künstlich eingefügte R-Schlag vor der Analyse aus dem EKG herausgeschnitten wird.

Unter "SVES-korrigiert-2" sind Spektralwerte angegeben, die ermittelt worden sind, nachdem das EKG wie oben beschrieben in der erfinderischen Art und Weise korrigiert worden ist.

Tabelle 1

|  | VLF [ms$^2$] | LF [ms$^2$] | HF [ms$^2$] | LF/HF |
|---|---|---|---|---|
| Referenz | 1753 | 1935 | 162 | 11,94 |
| SVES-Referenz | 1748 | 2087 | 732 | 2,85 |
| SVES-korrigiert-1 | 1768 | 1580 | 181 | 8,73 |
| SVES-korrigiert-2 | 1742 | 1902 | 159 | 11,96 |

Wie sich den Werten von "Referenz" und "SVES-Referenz" entnehmen lässt, wirkt sich eine einzige supraventrikulären Extrasystole stark auf die Spektralwerte aus, und eine Bestimmung der Herzfrequenzvariabilität würde erheblich verfälscht.

Ferner ist erkennbar, dass auch die Korrektur durch Wegschneiden (Spektralwerte unter "SVES-korregiert-1") zu großen Abweichungen führen und eine korrekte Bestimmung der Herzfrequenzvariabilität nicht zulassen.

Es versteht sich, dass bei Auftreten mehrerer SVES während der Messung noch größere Abweichungen zu erwarten wären.

Wird dagegen die Korrektur des EKGs in der oben beschriebenen erfindungsgemäßen Art und Weise durchgeführt, wird trotz des im EKG vorhandenen ungeeigneten R-Ausschlags ein Ergebnis erzielt, das mit der ursprünglichen Referenzanalyse nahezu übereinstimmt.

**Patentansprüche**

1. Verfahren zur Bestimmung der Herzfrequenzvariabilität eines Lebewesens, insbesondere eines Menschen, bei dem mittels einer Messeinrichtung Herzschläge des Lebewesens und Intervalle zwischen den Herzschlägen in Abhängigkeit von der Zeit als ein Messergebnis bestimmt werden, die Intervalle mittels schneller Fourier-Transformation (Fast Fourier Transformation) analysiert werden und ein Frequenzspektrum erstellt wird, bei dem vor Analyse mittels der Fourier-Transformation eine Prüfung der bestimmten Intervalle durchgeführt wird und das Messergebnis in Abhängigkeit von einem Ergebnis der Prüfung bearbeitet wird, wobei bei der Prüfung der ermittelten Intervalle geprüft wird, ob die Herzschläge zur Bestimmung der Herzfrequenzvariabilität geeignet sind, und zumindest einer der Herzschläge, die zur Bestimmung der Herzfrequenzvariabilität ungeeignet sind, aus dem Messergebnis gelöscht wird und durch einen künstlichen Herzschlag ersetzt wird, der im Messergebnis eine aufgrund zumindest einer der Herzschläge vor und nach dem ungeeigneten Herzschlag zu erwartende zeitliche Anordnung aufweist, **dadurch gekennzeichnet, dass** die zeitliche Anordnung des künstlichen Herzschlags davon abhängig vorgesehen wird, ob der ungeeignete Herzschlag zu einem Zeitpunkt stattgefunden hat, zu dem die Frequenz der Herzschläge wegen respiratorischer Sinusarrhythmie (RSA) steigend oder sinkend war.

2. Verfahren nach Anspruch 1,
   **dadurch gekennzeichnet,**
   **dass** aus zumindest je drei, vorzugsweise aus zumindest je vier, der Intervalle zwischen den Herzschlägen vor und nach dem ungeeigneten Herzschlag interpoliert wird.

3. Verfahren nach Anspruch 1 oder 2,
   **dadurch gekennzeichnet,**
   **dass** der künstliche Herzschlag im Messergebnis einen gleich großen oder im Wesentlichen gleich großen Zeitabschnitt einnimmt wie der ungeeignete Herzschlag.

4. Verfahren nach einem der Ansprüche 1 bis 3,
   **dadurch gekennzeichnet,**
   **dass** die zeitliche Anordnung des künstlichen Herzschlags mittels Interpolation der Herzschläge vor und nach dem ungeeigneten Herzschlag bestimmt wird.

5. Verfahren nach einem der Ansprüche 1 bis 4,
   **dadurch gekennzeichnet,**
   **dass** der künstliche Herzschlag bei steigender Frequenz vorzugsweise in einem zeitlich kleineren Abstand zu dem Herzschlag vor dem ungeeigneten Herzschlag als zu dem Herzschlag nach dem ungeeigneten Herzschlag vorgesehen wird.

6. Verfahren nach einem der Ansprüche 1 bis 5,
   **dadurch gekennzeichnet,**
   **dass** die ermittelten Intervalle zwischen den Herzschlägen durch Neuabtasten in eine Zeitreihe mit äquidistanten Intervallabständen gewandelt werden.

7. Verfahren nach Anspruch 6,
   **dadurch gekennzeichnet,**
   **dass** zur Neuabtastung eine auf kubischen Splines basierende Interpolationsmethode angewandt wird.

**8.** Verfahren nach Anspruch 7,
**dadurch gekennzeichnet,**
**dass** bei der Neuabtastung eine Integrationsbreite derart gewählt wird, dass die Anzahl der Intervalle zwischen den Herzschlägen gleich einer Zweierpotenz ($2^n$) ist.

**9.** Verfahren nach einem der Ansprüche 6 bis 8,
**dadurch gekennzeichnet,**
**dass** von den ermittelten Intervallen zwischen den Herzschlägen ein linearer oder parabolischer Trend subtrahiert wird, der durch Änderung der Frequenz der Herzschläge hervorgerufen wird.

**10.** Verfahren nach einem der Ansprüche 1 bis 9,
**dadurch gekennzeichnet,**
**dass** die ermittelten Intervalle durch Multiplikation mit einer Funktion zur Gewichtung, vorzugsweise einer Hann-, Hamming-, Kaiser-Bessel- oder Gauß-Fensterfunktion, gewichtet werden.

**11.** Verfahren nach einem der Ansprüche 1 bis 10,
**dadurch gekennzeichnet,**
**dass** zur Bestimmung der Herzschläge erste positive Ausschläge einer Herzkammerregung (R-Ausschläge) ermittelt werden und/oder als die Messeinrichtung ein Herzfrequenzmessgerät, vorzugsweise ein Brustgurt oder ein optischer Sensor z.B. zur Messung am Finger, oder ein EKG-Gerät verwendet wird.

**12.** Vorrichtung zur Bestimmung der Herzfrequenzvariabilität eines Lebewesens, insbesondere eines Menschen, die eine Messeinrichtung zur Bestimmung von Herzschlägen des Lebewesens und Intervallen zwischen den Herzschlägen in Abhängigkeit von der Zeit und eine Einrichtung zur Analyse der Intervalle mittels schneller Fourier-Transformation (Fast Fourier Transformation) und zur Erstellung eines Frequenzspektrums aufweist, wobei vor Analyse mittels der Fourier-Transformation eine Prüfung der bestimmten Intervalle durchgeführt wird und das Messergebnis in Abhängigkeit von einem Ergebnis der Prüfung vorgesehen ist,
dass die Vorrichtung dazu eingerichtet ist, bei der Prüfung der ermittelten Intervalle zu überprüfen, ob die Herzschläge zur Bestimmung der Herzfrequenzvariabilität geeignet sind, und zumindest einer der Herzschläge, die zur Bestimmung der Herzfrequenzvariabilität ungeeignet sind, aus dem Messergebnis zu löschen und durch einen künstlichen Herzschlag zu ersetzen, der im Messergebnis eine aufgrund zumindest einer der Herzschläge vor und nach dem ungeeigneten Herzschlag zu erwartende zeitliche Anordnung aufweist, **dadurch gekennzeichnet, dass** die zeitliche Anordnung des künstlichen Herzschlags davon abhängig vorgesehen wird, ob der ungeeignete Herzschlag zu einem Zeitpunkt stattgefunden hat, zu dem die Frequenz der Herzschläge wegen respiratorischer Sinusarrhythmie (RSA) steigend oder sinkend war.

## Claims

**1.** Method for determining the heart rate variability of a living being, especially of a human, in which heartbeats of the living being and intervals between the heartbeats as a function of time are determined as a measurement result by means of a measurement device, the intervals are analysed by means of fast Fourier transformation and a frequency spectrum is created, in which a check of the determined intervals is carried out before the Fourier transformation analysis and the measurement result is revised depending on a result of the check, wherein the check of the ascertained intervals involves checking whether the heartbeats are suitable for determining the heart rate variability and at least one of the heartbeats unsuitable for determining the heart rate variability is deleted from the measurement result and is replaced with an artificial heartbeat which has in the measurement result a temporal arrangement to be expected on the basis of at least one of the heartbeats before and after the unsuitable heartbeat,
**characterized in that**
the temporal arrangement of the artificial heartbeat is provided dependent on whether the unsuitable heartbeat took place at a time point at which the frequency of the heartbeats was rising or falling because of respiratory sinus arrhythmia (RSA).

**2.** Method according to Claim 1,
**characterized in that**
interpolation is carried out from at least three in each case, preferably from at least four in each case, of the intervals between the heartbeats before and after the unsuitable heartbeat.

3. Method according to Claim 1 or 2,
**characterized in that**
the artificial heartbeat occupies in the measurement result a same-sized or substantially same-sized time period as the unsuitable heartbeat.

4. Method according to any of Claims 1 to 3,
**characterized in that**
the temporal arrangement of the artificial heartbeat is determined by means of interpolation of the heartbeats before and after the unsuitable heartbeat.

5. Method according to any of Claims 1 to 4,
**characterized in that**
the artificial heartbeat is, in the case of a rising frequency, preferably provided at a temporally smaller distance in relation to the heartbeat before the unsuitable heartbeat than in relation to the heartbeat after the unsuitable heartbeat.

6. Method according to any of Claims 1 to 5,
**characterized in that**
the ascertained intervals between the heartbeats are converted by resampling into a time series having equidistant interval distances.

7. Method according to Claim 6,
**characterized in that**
a cubic spline-based interpolation method is used for the resampling.

8. Method according to Claim 7,
**characterized in that**
an integration width is chosen in the resampling such that the number of intervals between the heartbeats is equal to a power of two ($2^n$).

9. Method according to any of Claims 6 to 8,
**characterized in that**
a linear or parabolic trend caused by a change in the frequency of the heartbeats is subtracted from the ascertained intervals between the heartbeats.

10. Method according to any of Claims 1 to 9,
**characterized in that**
the ascertained intervals are weighted by multiplication with a function for weighting, preferably a Hann, Hamming, Kaiser-Bessel or Gaussian window function.

11. Method according to any of Claims 1 to 10,
**characterized in that**
the heartbeats are determined by ascertaining first positive deflections of a ventricle stimulation (R deflections) and/or using a heart rate monitor, preferably a chest belt or an optical sensor, for example for finger measurement, or an ECG instrument, as the measurement device.

12. Apparatus for determining the heart rate variability of a living being, especially of a human, comprising a measurement device for determining heartbeats of the living being and intervals between the heartbeats as a function of time and a device for analysing the intervals by means of fast Fourier transformation and for creating a frequency spectrum, wherein a check of the determined intervals is carried out before the Fourier transformation analysis and the measurement result is provided depending on a result of the check,
wherein the apparatus is configured to check, in the check of the ascertained intervals, whether the heartbeats are suitable for determining the heart rate variability and to delete at least one of the heartbeats unsuitable for determining the heart rate variability from the measurement result and to replace it with an artificial heartbeat which has in the measurement result a temporal arrangement to be expected on the basis of at least one of the heartbeats before and after the unsuitable heartbeat,
**characterized in that**
the temporal arrangement of the artificial heartbeat is provided dependent on whether the unsuitable heartbeat took place at a time point at which the frequency of the heartbeats was rising or falling because of respiratory sinus arrhythmia (RSA).

**Revendications**

1. Procédé pour déterminer la variabilité de la fréquence cardiaque d'un être vivant, en particulier d'un être humain, dans lequel
on détermine en tant que résultat de mesure au moyen d'un appareil de mesure les battements cardiaques de l'être vivant et les intervalles entre les battements cardiaques en fonction du temps,
on analyse les intervalles au moyen d'une transformation de Fourier rapide (Fast Fourier Transformation) et on établit un spectre de fréquences dans lequel, avant l'analyse au moyen de la transformation de Fourier,
on effectue une vérification des intervalles déterminés et on traite le résultat de mesure en fonction d'un résultat de la vérification, et
lors de la vérification des intervalles déterminés, on vérifie si les battements cardiaques sont appropriés pour déterminer la variabilité de la fréquence cardiaque, et
au moins l'un des battements cardiaques qui ne sont pas appropriés pour déterminer la variabilité de la fréquence cardiaque est supprimé du résultat de mesure et est remplacé par un battement cardiaque artificiel qui, dans le résultat de mesure, présente une disposition temporelle à laquelle il faut s'attendre en raison d'au moins l'un des battements cardiaques avant et après le battement cardiaque inapproprié,
**caractérisé en ce que**
la disposition temporelle du battement cardiaque artificiel est prévue en fonction de savoir si le battement cardiaque inapproprié s'est produit à un instant où la fréquence des battements cardiaques augmentait ou diminuait en raison d'une arythmie respiratoire sinusale (ARS).

2. Procédé selon la revendication 1,
**caractérisé en ce que**
il est prévu une interpolation à partir d'au moins trois, de préférence à partir d'au moins quatre desdits intervalles, entre les battements cardiaques avant et après le battement cardiaque inapproprié.

3. Procédé selon la revendication 1 ou 2,
**caractérisé en ce que**
dans le résultat de mesure, le battement cardiaque artificiel occupe une période de temps qui est égale ou essentiellement égale au battement cardiaque inapproprié.

4. Procédé selon l'une des revendications 1 à 3,
**caractérisé en ce que**
la disposition temporelle du battement cardiaque artificiel est déterminée par interpolation des battements cardiaques avant et après le battement cardiaque inapproprié.

5. Procédé selon l'une des revendications 1 à 4,
**caractérisé en ce que**
le battement cardiaque artificiel est prévu à une fréquence croissante, de préférence à une distance temporellement plus courte du battement cardiaque précédant le battement cardiaque inapproprié que du battement cardiaque suivant le battement cardiaque inapproprié.

6. Procédé selon l'une des revendications 1 à 5,
**caractérisé en ce que**
les intervalles déterminés entre les battements cardiaques sont convertis par rééchantillonnage en une série chronologique avec des distances d'intervalle équidistantes.

7. Procédé selon la revendication 6,
**caractérisé en ce que**
une méthode d'interpolation basée sur des splines cubiques est appliquée pour le rééchantillonnage.

8. Procédé selon la revendication 7,
**caractérisé en ce que**
pour le rééchantillonnage, une largeur d'intégration est choisie de sorte que le nombre d'intervalles entre les battements cardiaques soit égal à une puissance de deux ($2^n$).

**9.** Procédé selon l'une des revendications 6 à 8,
**caractérisé en ce que**
une tendance linéaire ou parabolique causée par changement dans la fréquence des battements cardiaques est soustraite des intervalles déterminés entre les battements cardiaques.

**10.** Procédé selon l'une des revendications 1 à 9,
**caractérisé en ce que**
les intervalles déterminés sont pondérés par multiplication avec une fonction de pondération, de préférence une fonction à fenêtre de Hann, Hammin, Kaiser-Bessel ou gaussienne.

**11.** Procédé selon l'une des revendications 1 à 10,
**caractérisé en ce que**
pour déterminer les battements cardiaques, on détermine des premières excursions positives d'une excitation ventriculaire (excursions R), et/ou on utilise à titre d'appareil de mesure un cardiofréquencemètre, de préférence une ceinture thoracique ou un capteur optique par exemple pour la mesure sur le doigt, ou un appareil ECG.

**12.** Dispositif pour déterminer la variabilité de la fréquence cardiaque d'un être vivant, en particulier d'un être humain, qui comprend un appareil de mesure pour déterminer les battements cardiaques de l'être vivant et les intervalles entre les battements cardiaques en fonction du temps, et un appareil destiné à analyser les intervalles au moyen d'une transformation de Fourier rapide (Fast Fourier Transformation) et à établir un spectre de fréquences, dans lequel, avant l'analyse au moyen de la transformation de Fourier, une vérification des intervalles déterminés est effectuée, et le résultat de mesure est prévu en fonction d'un résultat de la vérification, et le dispositif est conçu pour vérifier, lors de la vérification des intervalles déterminés, si les battements cardiaques sont appropriés pour déterminer la variabilité de la fréquence cardiaque, et au moins l'un des battements cardiaques qui ne sont pas appropriés pour déterminer la variabilité de la fréquence cardiaque est supprimé du résultat de mesure et est remplacé par un battement cardiaque artificiel qui, dans le résultat de mesure, présente une disposition temporelle à laquelle il faut s'attendre en raison d'au moins l'un des battements cardiaques avant et après le battement cardiaque inapproprié,
**caractérisé en ce que**
la disposition temporelle du battement cardiaque artificiel est prévue en fonction de savoir si le battement cardiaque inapproprié s'est produit à un instant où la fréquence des battements cardiaques augmentait ou diminuait en raison d'une arythmie respiratoire sinusale (ARS).

Fig. 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2011113428 A2
- US 20090005696 A1
- US 20100042172 A1
- US 20110105926 A1